# EUROPEAN PATENT APPLICATION

(11) **EP 4 627 996 A1**
(43) Date of publication of application: **08.10.2025**
(21) Application number: 23898090.8
(22) Date of filing: 31.10.2023
(51) Int. Cl.: A61B 5/00, G16H 50/20, G16H 50/50, G06N 3/08, A61B 5/01, A61B 5/021, A61B 5/08, A61B 5/024

(54) **METHOD FOR PREDICTING BIO EVENT**

(30) Priority: 30.11.2022 KR 20220163845; 19.09.2023 KR 20230124578
(71) Applicant: VUNO INC., Seoul 06541 (KR)
(72) Inventor: CHOI, Jaewoo, Seoul 06227 (KR); CHO, Kyungjae, Seongnam-si, Gyeonggi-do 13645 (KR); SHIN, Yunseob, Hwaseong-si, Gyeonggi-do 18479 (KR); TAE, Yunwon, Anyang-si, Gyeonggi-do 13994 (KR)
(74) Representative: Buzzi, Notaro & Antonielli d'Oulx S.p.A.
(86) International application number: PCT/KR2023/017125
(87) International publication number: WO 2024/117557

(57) **Abstract**

According to an embodiment of the present disclosure, disclosed is a method for predicting a bio event. Specifically, according to the present disclosure, a computing device obtains bio information of a patient; and generates a first output indicating an expected time of occurrence of the bio event based on the bio information by using a pre-trained artificial neural network model.

## Description

### [Technical Field]

The present disclosure relates to a method for predicting a bio event, and particularly, to a method for predicting a time of occurrence of a bio event, for example, an event related to a disease that is predetermined to have a fatal impact on a health status of a patient, based on patient's biological information, by utilizing an artificial neural network model.

### [Background Art]

Methods for predicting potential patient events, such as a cardiac arrest, from patient biometric information including body temperature, blood pressure, respiratory rate, heart rate, and electrocardiogram, and performing intensive monitoring and taking necessary measures in advance have been used in a medical field.

In particular, recently, a method for predicting a patient's bio event using a deep learning model, a branch of machine learning, is being studied. Machine learning models that perform these functions operate by taking in multiple inputs and producing a single output value using recurrent neural networks, long short-term memory (LSTM), etc.

However, a conventional method for predicting the patient's bio event using the deep learning model provide only very limited information, such as a possibility that a patient will experience a bio event such as cardiac arrest within a predetermined period of time. In this case, if there are multiple patients with a potential for occurrence of various types of bio events, it is difficult to know which one is more critical and to take necessary measures depending on a critical condition.

Therefore, there is a need in the art for the method for predicting the bio event so that necessary interventions can be taken in more critically ill patients.

Korean Patent Unexamined Publication No. KR 2022-0040525 A discloses a system for predicting a degree of risk of a cardiac arrest by using an electrocardiogram based on deep learning.

### [Disclosure]

### [Technical Problem]

The present disclosure is contrived in response to the aforementioned background technology, and has been made in an effort to predict a bio event by having an artificial neural network model generate a first output related to an expected time of occurrence of the bio event based on a patient's bio information input.

Meanwhile, a technical object to be achieved by the present disclosure is not limited to the above-mentioned technical object, and various technical objects can be included within the scope which is apparent to those skilled in the art from contents to be described below.

### [Technical Solution]

In order to implement the object described above, disclosed is a method performed by a computing device to predict a bio event in a medical field according to an embodiment of the present disclosure. The method include: obtaining bio information of a patient; and generating a first output indicating an expected time of occurrence of the bio event based on the bio information by using an artificial neural network model.

In an embodiment, the bio event may include an event related to a disease determined to affect a health status of the patient.

In an embodiment, the method may further include generating a second output indicating a possibility of occurrence of the bio event within a predetermined time period by using the artificial neural network model.

In an embodiment, the first output may include a value corresponding to a length of a remaining time from a time when the bio information is obtained up to a time when the bio event is predicted to occur.

In an embodiment, the method may further include providing, to a user, comprehensive prediction information related to the bio event based on the first output and the second output, and the comprehensive prediction information may include information capable of determining a priority for each patient among all patients.

In an embodiment, the comprehensive prediction information may include information through a comparison between the second output and a predetermined threshold, and priority information of the patient determined based on the filtered result and a size of the first output.

In order to implement the object described above, according to an embodiment of the present disclosure, disclosed is a method performed by a computing device to predict a bio event. The method may include: obtaining bio information of a patient; and generating prediction information related to a bio event based on the bio information by using an artificial neural network model, and the artificial neural network model may be a model trained by multi-task learning based on a first task of predicting the expected time of occurrence of the bio event and a second task of predicting the probability of occurrence of the bio event.

In an embodiment, the artificial neural network model may be trained based on two or more different training data sets.

In an embodiment, the two or more different training data sets may include first training data which is bio information with information on the occurrence time of the bio event of the patient set as a label.

In an embodiment, the two or more different training data sets may include second training data which is bio information with binary information indicating whether the bio event occurs within a predetermined time period set as a label.

In order to implement the object described above, according to an embodiment of the present disclosure, disclosed is a computer program stored in a computer readable storage medium, which includes instructions which allow a computing device to perform operations of predicting a bio event. The operations may include: an operation of obtaining bio information of a patient; and an operation of generating a first output indicating an expected time of occurrence of the bio event based on the bio information by using an artificial neural network model.

In order to implement the object described above, according to an embodiment of the present disclosure, disclosed is a computing device for predicting a bio event. The computing device may include: at least one processor; and a memory, and the one or more processors may obtain bio information of a patient, and generate a first output indicating an expected time of occurrence of the bio event based on the bio information by using an artificial neural network model.

### [Advantageous Effects]

The present disclosure can provide bio event prediction information that can aid in the treatment of patients in whom a bio event has occurred or has not yet occurred. For example, the present disclosure can generate multiple types of prediction information of different types related to a bio event based on a patient's bio information, and can optimally perform treatment for a patient in whom a bio event has occurred or has not yet occurred based on the generated multiple pieces of prediction information.

Meanwhile, a technical object to be achieved by the present disclosure is not limited to the above-mentioned technical object, and various technical objects can be included within the scope which is apparent to those skilled in the art from contents to be described below.

### [Description of Drawings]

The following accompanying drawings are only some of the embodiment of the present disclosure so as to be used for describing the embodiment of the present disclosure, and in the technical field of the present disclosure, those skilled in the technical field of the present disclosure can obtain other drawings based on the drawings without an effort to reach a new invention.
FIG. 1 is a block diagram of a computing device for predicting a bio event according to an embodiment of the present disclosure.
FIG. 2 is a schematic diagram illustrating a network function according to an exemplary embodiment of the present disclosure.
FIG. 3 is a flowchart illustrating a process for predicting a bio event according to an embodiment of the present disclosure.
FIGS. 4A and 4B are conceptual diagrams illustrating a label of first training data according to an embodiment of the present disclosure.
FIG. 4C is a conceptual diagram illustrating a label of second training data according to an embodiment of the present disclosure.
FIG. 5 is a simple and general schematic diagram illustrating an example of a computing environment in which the embodiments of the present disclosure are implementable.

### [Best Mode]

The present disclosure discloses a method for predicting a bio event through a first output indicating an expected time of occurrence of the bio event and a second output indicating a possibility of occurrence of the bio event based on a patient's bio information by utilizing an artificial neural network model.

Various exemplary embodiments will now be described with reference to drawings. In the present specification, various descriptions are presented to provide appreciation of the present disclosure. However, it is apparent that the exemplary embodiments can be executed without the specific description.

"Component", "module", "system", and the like which are terms used in the specification refer to a computer-related entity, hardware, firmware, software, and a combination of the software and the hardware, or execution of the software. For example, the component may be a processing procedure executed on a processor, the processor, an object, an execution thread, a program, and/or a computer, but is not limited thereto. For example, both an application executed in a computing device and the computing device may be the components. One or more components may reside within the processor and/or a thread of execution. One component may be localized in one computer. One component may be distributed between two or more computers. Further, the components may be executed by various computer-readable media having various data structures, which are stored therein. The components may perform communication through local and/or remote processing according to a signal (for example, data transmitted from another system through a network such as the Internet through data and/or a signal from one component that interacts with other components in a local system and a distribution system) having one or more data packets, for example.

The term "or" is intended to mean not exclusive "or" but inclusive "or". That is, when not separately specified or not clear in terms of a context, a sentence "X uses A or B" is intended to mean one of the natural inclusive substitutions. That is, the sentence "X uses A or B" may be applied to any of the case where X uses A, the case where X uses B, or the case where X uses both A and B. Further, it should be understood that the term "and/or" used in this specification designates and includes all available combinations of one or more items among enumerated related items.

It should be appreciated that the term "comprise" and/or "comprising" means presence of corresponding features and/or components. However, it should be appreciated that the term "comprises" and/or "comprising" means that presence or addition of one or more other features, components, and/or a group thereof is not excluded. Further, when not separately specified or it is not clear in terms of the context that a singular form is indicated, it should be construed that the singular form generally means "one or more" in this specification and the claims.

The term "at least one of A or B" should be interpreted to mean "a case including only A", "a case including only B", and "a case in which A and B are combined".

Those skilled in the art need to recognize that various illustrative logical blocks, configurations, modules, circuits, means, logic, and algorithm steps described in connection with the exemplary embodiments disclosed herein may be additionally implemented as electronic hardware, computer software, or combinations of both sides. To clearly illustrate the interchangeability of hardware and software, various illustrative components, blocks, configurations, means, logic, modules, circuits, and steps have been described above generally in terms of their functionalities. Whether the functionalities are implemented as the hardware or software depends on a specific application and design restrictions given to an entire system. Skilled artisans may implement the described functionalities in various ways for each particular application. However, such implementation decisions should not be interpreted as causing a departure from the scope of the present disclosure.

The description of the presented exemplary embodiments is provided so that those skilled in the art of the present disclosure use or implement the present disclosure. Various modifications to the exemplary embodiments will be apparent to those skilled in the art. Generic principles defined herein may be applied to other embodiments without departing from the scope of the present disclosure. Therefore, the present disclosure is not limited to the exemplary embodiments presented herein. The present disclosure should be analyzed within the widest range which is coherent with the principles and new features presented herein.

In the present disclosure, a 'bio event' may mean a disease-related event that is predetermined to affect the health status of a patient. For example, the bio event may include critical events such as cardiac arrest, sepsis, stroke, arrhythmia, myocardial infarction, heart failure, and unplanned ICU admission.

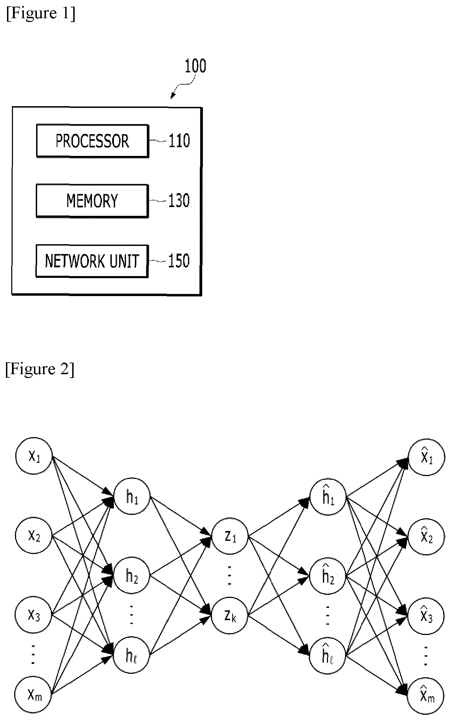

FIG. 1 is a block diagram of a computing device for predicting a bio event according to an embodiment of the present disclosure.

A configuration of the computing device 100 illustrated in FIG. 1 is only an example shown through simplification. In an embodiment of the present disclosure, the computing device 100 may include other components for performing the computing environment of the computing device 100, and only some of the disclosed components may constitute the computing device 100.

The computing device 100 may include a processor 110, a memory 130, and a network unit 150.

The processor 110 may be constituted by one or more cores and may include processors for data analysis and deep learning, which include a central processing unit (CPU), a general purpose graphics processing unit (GPGPU), a tensor processing unit (TPU), and the like of the computing device. The processor 110 may read a computer program stored in the memory 130 to perform data processing for machine learning according to an exemplary embodiment of the present disclosure. According to an exemplary embodiment of the present disclosure, the processor 110 may perform a calculation for learning the neural network. The processor 110 may perform calculations for learning the neural network, which include processing of input data for learning in deep learning (DL), extracting a feature in the input data, calculating an error, updating a weight of the neural network using backpropagation, and the like.

At least one of the CPU, GPGPU, and TPU of the processor 110 may process learning of a network function. For example, both the CPU and the GPGPU may process the learning of the network function and data classification using the network function. Further, in an exemplary embodiment of the present disclosure, processors of a plurality of computing devices may be used together to process the learning of the network function and the data classification using the network function. Further, the computer program executed in the computing device according to an exemplary embodiment of the present disclosure may be a CPU, GPGPU, or TPU executable program.

According to an embodiment of the present disclosure, the processor 110 may obtain bio information of a patient. At this time, a path for obtaining the bio information may be data stored in the memory 130 of the computing device may be information measured from a biometric information measurement device connected to the computing device in real time, and may be information received from the network unit 150. However, the present disclosure is not limited to such an acquisition path.

The bio information may include various bio-related signals such as the patient's body temperature, blood pressure, respiration rate, heart rate, electrocardiogram (ECG), electromyogram (EMG), electroencephalogram (EEG), electrodermal activity (EDA), skin temperature (SKT), photoplethysmography (PPG), X-ray imaging signals, magnetic resonance imaging (MRI) signals, and computed tomography (CT) imaging signals. Further, the bio information is not limited to these examples and may include various information obtained from a subject using medical devices, wearable devices, mobile devices, etc.

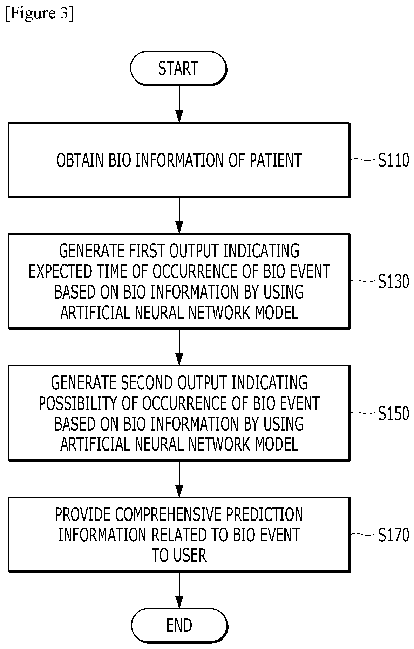

The processor 110 may generate a first output indicating an expected time of occurrence of a bio event based on the bio information by utilizing a pre-trained artificial neural network model. A specific method of generating the first output will be described later with reference to FIG. 3.

In the present disclosure, the bio event includes an event related to a disease that affects a health status of a patient, and collectively refers to symptoms, disease names, etc., that have clinically significant meaning, and in an embodiment, the bio event may mean cardiac arrest, sepsis, myocardial infarction, heart failure, arrhythmia, etc. However, as with the bio information, the bio event is not to be construed as being limited to what is exemplarily described in the present disclosure.

In an embodiment, an artificial neural network model may generate, as a first output, an expected time of occurrence of cardiac arrest or sepsis based on a vital sign of the patient. In another embodiment, the artificial neural network model may generate, as the first output, an expected time of occurrence of major cardiovascular diseases such as myocardial infarction, heart failure, arrhythmia, chronic kidney disease, and hyperkalemia based on the patient's electrocardiogram (ECG).

The processor 110 may generate a second output indicating a possibility of occurrence of the bio event based on the bio information by utilizing a pre-trained artificial neural network model. A specific method of generating the second output will be described later with reference to FIG. 3.

In an embodiment, the artificial neural network model may generate, as the second output, the possibility of occurrence of cardiac arrest or sepsis based on the vital sign of the patient. In another embodiment, the artificial neural network model may generate, as the second output, a possibility of occurrence of major cardiovascular diseases such as myocardial infarction, heart failure, arrhythmia, chronic kidney disease, and hyperkalemia based on the patient's electrocardiogram (ECG).

The processor 110 may provide, to a user, comprehensive prediction information related to the bio event based on the first output and the second output. The comprehensive prediction information may serve as a criterion for determining the level of monitoring necessary for a patient having the possibility of occurrence of the bio event.

For example, the processor 110 may provide the comprehensive prediction information to the user by predicting patients likely to have cardiac arrest within a predetermined time through the second output and primarily filtering the patients, and sorting the patients in order of the expected time of cardiac arrest among the patients through the first output.

As another example, the processor 110 may output the occurrence probability and expected occurrence time of multiple types of biological events and provide the comprehensive prediction information to the user by simultaneously considering the fatality and expected occurrence time of each bio event.

By providing, to the user, the comprehensive prediction information related to the bio event in the present disclosure, the frequency or amount of information provided to a human physician, such as alarms, increases, so that higher intensity monitoring may be performed efficiently.

According to an exemplary embodiment of the present disclosure, the memory 130 may include at least one type of storage medium of a flash memory type storage medium, a hard disk type storage medium, a multimedia card micro type storage medium, a card type memory (for example, an SD or XD memory, or the like), a random access memory (RAM), a static random access memory (SRAM), a read-only memory (ROM), an electrically erasable programmable read-only memory (EEPROM), a programmable read-only memory (PROM), a magnetic memory, a magnetic disk, and an optical disk. The computing device 100 may operate in connection with a web storage performing a storing function of the memory 130 on the Internet. The description of the memory is just an example and the present disclosure is not limited thereto.

The network unit 150 according to several embodiments of the present disclosure may use various wired communication systems, such as a Public Switched Telephone Network (PSTN), an x Digital Subscriber Line (xDSL), a Rate Adaptive DSL (RADSL), a Multi Rate DSL (MDSL), a Very High Speed DSL (VDSL), a Universal Asymmetric DSL (UADSL), a High Bit Rate DSL (HDSL), and a local area network (LAN).

The network unit 150 presented in the present specification may use various wireless communication systems, such as Code Division Multi Access (CDMA), Time Division Multi Access (TDMA), Frequency Division Multi Access (FDMA), Orthogonal Frequency Division Multi Access (OFDMA), Single Carrier-FDMA (SC-FDMA), and other systems.

The network unit 150 according to an exemplary embodiment of the present disclosure may use an arbitrary type known wired/wireless communication systems.

The techniques described herein may be used in other networks in addition to those mentioned above.

FIG. 2 is a schematic diagram illustrating a network function according to an exemplary embodiment of the present disclosure.

Throughout the present specification, a computation model, the neural network, a network function, and the neural network may be used as the same meaning. The neural network may be generally constituted by an aggregate of calculation units which are mutually connected to each other, which may be called nodes. The nodes may also be called neurons. The neural network is configured to include one or more nodes. The nodes (alternatively, neurons) constituting the neural networks may be connected to each other by one or more links.

**In** the neural network, one or more nodes connected through the link may relatively form the relationship between an input node and an output node. Concepts of the input node and the output node are relative and a predetermined node which has the output node relationship with respect to one node may have the input node relationship in the relationship with another node and vice versa. As described above, the relationship of the input node to the output node may be generated based on the link. One or more output nodes may be connected to one input node through the link and vice versa.

In the relationship of the input node and the output node connected through one link, a value of data of the output node may be determined based on data input in the input node. Here, a link connecting the input node and the output node to each other may have a weight. The weight may be variable and the weight is variable by a user or an algorithm in order for the neural network to perform a desired function. For example, when one or more input nodes are mutually connected to one output node by the respective links, the output node may determine an output node value based on values input in the input nodes connected with the output node and the weights set in the links corresponding to the respective input nodes.

As described above, in the neural network, one or more nodes are connected to each other through one or more links to form a relationship of the input node and output node in the neural network. A characteristic of the neural network may be determined according to the number of nodes, the number of links, correlations between the nodes and the links, and values of the weights granted to the respective links in the neural network. For example, when the same number of nodes and links exist and there are two neural networks in which the weight values of the links are different from each other, it may be recognized that two neural networks are different from each other.

The neural network may be constituted by a set of one or more nodes. A subset of the nodes constituting the neural network may constitute a layer. Some of the nodes constituting the neural network may constitute one layer based on the distances from the initial input node. For example, a set of nodes of which distance from the initial input node is n may constitute n layers. The distance from the initial input node may be defined by the minimum number of links which should be passed through for reaching the corresponding node from the initial input node. However, a definition of the layer is predetermined for description and the order of the layer in the neural network may be defined by a method different from the aforementioned method. For example, the layers of the nodes may be defined by the distance from a final output node.

The initial input node may mean one or more nodes in which data is directly input without passing through the links in the relationships with other nodes among the nodes in the neural network. Alternatively, in the neural network, in the relationship between the nodes based on the link, the initial input node may mean nodes which do not have other input nodes connected through the links. Similarly thereto, the final output node may mean one or more nodes which do not have the output node in the relationship with other nodes among the nodes in the neural network. Further, a hidden node may mean nodes constituting the neural network other than the initial input node and the final output node.

In the neural network according to an exemplary embodiment of the present disclosure, the number of nodes of the input layer may be the same as the number of nodes of the output layer, and the neural network may be a neural network of a type in which the number of nodes decreases and then, increases again from the input layer to the hidden layer. Further, in the neural network according to another exemplary embodiment of the present disclosure, the number of nodes of the input layer may be smaller than the number of nodes of the output layer, and the neural network may be a neural network of a type in which the number of nodes decreases from the input layer to the hidden layer. Further, in the neural network according to yet another exemplary embodiment of the present disclosure, the number of nodes of the input layer may be larger than the number of nodes of the output layer, and the neural network may be a neural network of a type in which the number of nodes increases from the input layer to the hidden layer. The neural network according to still yet another exemplary embodiment of the present disclosure may be a neural network of a type in which the neural networks are combined.

A deep neural network (DNN) may refer to a neural network that includes a plurality of hidden layers in addition to the input and output layers. When the deep neural network is used, the latent structures of data may be determined. That is, latent structures of photos, text, video, voice, and music (e.g., what objects are in the photo, what the content and feelings of the text are, what the content and feelings of the voice are) may be determined. The deep neural network may include a convolutional neural network (CNN), a recurrent neural network (RNN), an auto encoder, generative adversarial networks (GAN), a restricted Boltzmann machine (RBM), a deep belief network (DBN), a Q network, a U network, a Siam network, a Generative Adversarial Network (GAN), and the like. The description of the deep neural network described above is just an example and the present disclosure is not limited thereto.

In an exemplary embodiment of the present disclosure, the network function may include the auto encoder. The auto encoder may be a kind of artificial neural network for outputting output data similar to input data. The auto encoder may include at least one hidden layer and odd hidden layers may be disposed between the input and output layers. The number of nodes in each layer may be reduced from the number of nodes in the input layer to an intermediate layer called a bottleneck layer (encoding), and then expanded symmetrical to reduction to the output layer (symmetrical to the input layer) in the bottleneck layer. The auto encoder may perform non-linear dimensional reduction. The number of input and output layers may correspond to a dimension after preprocessing the input data. The auto encoder structure may have a structure in which the number of nodes in the hidden layer included in the encoder decreases as a distance from the input layer increases. When the number of nodes in the bottleneck layer (a layer having a smallest number of nodes positioned between an encoder and a decoder) is too small, a sufficient amount of information may not be delivered, and as a result, the number of nodes in the bottleneck layer may be maintained to be a specific number or more (e.g., half of the input layers or more).

The neural network may be trained in at least one scheme of supervised learning, unsupervised learning, semi supervised learning, or reinforcement learning. The training of the neural network may be a process in which the neural network applies knowledge for performing a specific operation to the neural network.

The neural network may be trained in a direction to minimize errors of an output. The training of the neural network is a process of repeatedly inputting training data into the neural network and calculating the output of the neural network for the training data and the error of a target and back-propagating the errors of the neural network from the output layer of the neural network toward the input layer in a direction to reduce the errors to update the weight of each node of the neural network. In the case of the supervised learning, the training data labeled with a correct answer is used for each training data (i.e., the labeled training data) and in the case of the unsupervised learning, the correct answer may not be labeled in each training data. That is, for example, the training data in the case of the supervised learning related to the data classification may be data in which category is labeled in each training data. The labeled training data is input to the neural network, and the error may be calculated by comparing the output (category) of the neural network with the label of the training data. As another example, in the case of the unsupervised learning related to the data classification, the training data as the input is compared with the output of the neural network to calculate the error. The calculated error is back-propagated in a reverse direction (i.e., a direction from the output layer toward the input layer) in the neural network and connection weights of respective nodes of each layer of the neural network may be updated according to the back propagation. A variation amount of the updated connection weight of each node may be determined according to a training rate. Calculation of the neural network for the input data and the back-propagation of the error may constitute a training cycle (epoch). The training rate may be applied differently according to the number of repetition times of the training cycle of the neural network. For example, in an initial stage of the training of the neural network, the neural network ensures a certain level of performance quickly by using a high training rate, thereby increasing efficiency and uses a low training rate in a latter stage of the training, thereby increasing accuracy.

In training of the neural network, the training data may be generally a subset of actual data (i.e., data to be processed using the trained neural network), and as a result, there may be a training cycle in which errors for the training data decrease, but the errors for the actual data increase. Overfitting is a phenomenon in which the errors for the actual data increase due to excessive training of the training data. For example, a phenomenon in which the neural network that trains a cat by showing a yellow cat sees a cat other than the yellow cat and does not recognize the corresponding cat as the cat may be a kind of overfitting. The overfitting may act as a cause which increases the error of the machine learning algorithm. Various optimization methods may be used in order to prevent the overfitting. In order to prevent the overfitting, a method such as increasing the training data, regularization, dropout of omitting a part of the node of the network in the process of training, utilization of a batch normalization layer, etc., may be applied.

FIG. 3 is a flowchart illustrating a process for predicting a bio event according to an embodiment of the present disclosure.

Referring to FIG. 3, the process of predicting the bio event may include a step (S110) of obtaining bio information of a patient, a step (S130) of generating a first output indicating an expected time of occurrence of the bio event based on the bio information by utilizing an artificial neural network model, a step (S150) of generating a second output indicating a possibility of occurrence of the bio event based on the bio information by utilizing the artificial neural network model, and a step (S170) of providing, to a user, comprehensive prediction information related to the bio event.

In step S110, the processor 110 may obtain the bio information of the patient. A specific method for obtaining the bio information of the patient is described above with reference to FIG. 1.

In step S130, the processor 110 may generate a first output indicating the expected time of occurrence of the bio event based on the bio information by utilizing the artificial neural network model. At this time, the first output may include a value corresponding to a length of time remaining until a time at which the bio event is expected to occur. For example, the first output may be designed to have a larger value the closer the patient is predicted to experience cardiac arrest. More specifically, the artificial neural network model may output 0 as the first output if the bio event is predicted to occur after 24 hours, 0.5 if the bio event is predicted to occur after 12 hours, and 0.75 if the bio event is predicted to occur after 6 hours. The user, for example a human doctor, may use the first output of the artificial neural network model to determine when the bio event is to occur in the patient.

In step S150, the processor 110 may generate a second output indicating a possibility of occurrence of the bio event based on the bio information by utilizing the artificial neural network model. At this time, the second output may include information indicating the possibility of occurrence of the bio event within a predetermined time period.

For example, the second output may be a value representing a probability that the patient will experience cardiac arrest within the predetermined time period as a real number between 0 and 1. In this case, the higher the possibility that cardiac arrest will occur within the predetermined time, the closer the second output may be represented as a value closer to 1, and if it is definitively predicted that cardiac arrest will occur, the second output may be 1.

Specifically, if the artificial neural network model is configured to output the probability of the patient experiencing cardiac arrest, and patient A has a higher probability of experiencing cardiac arrest within a predetermined time period than patient B, the second output of the artificial neural network model for patient A may be set to have a higher value than the second output of the artificial neural network model for patient B. In this case, the second output value for patient A may be 0.8, and the second output value for patient B may be 0.2.

In step S170, the processor 110 may provide, to the user, the comprehensive prediction information related to the bio event. At this time, the comprehensive prediction information may be information about the likelihood that a bio event will occur within a predetermined time period for a patient, and if it is predicted to occur, how much time is predicted to elapse before the bio event occurs.

For example, the processor 110 may primarily filter patients in which a possibility of occurrence of cardiac arrest within a predetermined time period is predicted to be equal to or more than a predetermined threshold among all patients through the second output, and then sort the patients in order of the expected time of cardiac arrest, and rank the sorted patients through the first output. And, the comprehensive prediction information may be provided to the user in a scheme that indicates a higher severity level for patients with higher rankings. Therefore, the above comprehensive prediction information can be used to determine the priority for each patient among all patients.

In the above example, the artificial neural network model is configured to output the possibility of the patient experiencing cardiac arrest, but in the present disclosure, the artificial neural network model may also be configured to receive the patient's bio information and generate outputs for different types of bio events. For example, the artificial neural network model of the present disclosure may include a first sub-model related to cardiac arrest and a second sub-model related to sepsis. In this case, the first sub-model may be an artificial neural network model that receives the patient's bio information and outputs the expected time and probability of the patient's experiencing cardiac arrest, and the second sub-model may be an artificial neural network model that receives the patient's bio information and outputs the expected time and probability of the patient's experiencing sepsis.

However, the types of bio events in the present disclosure are not limited to the above examples, and when generating outputs for different types of bio events, the artificial neural network model may be an artificial neural network model configured to generate outputs for different types of bio events by applying a multi-task learning method.

Hereinafter, the artificial neural network model that generates the prediction information related to the bio event based on the bio information is described.

In the present disclosure, the artificial neural network model that generates the prediction information related to the bio event based on the bio information may be a machine learning model, more specifically, a deep learning model.

More specifically, the artificial neural network model of the present disclosure may include a neural network such as a recurrent neural network (RNN), a long-shot term memory (LSTM), and a transformer. However, an appropriate form of neural network may be added or removed to configure the artificial neural network model of the present disclosure, and the present disclosure is not limited to the type of neural network model exemplified above.

The artificial neural network model of the present disclosure may receive multiple types of inputs simultaneously. Specifically, the artificial neural network model of the present disclosure may simultaneously receive multiple types of bio information from among human bio information and generate one or more outputs. More specifically, the artificial neural network model can have a 'many-to-one' structure that takes the patient's body temperature, blood pressure, respiratory rate, heart rate, and electrocardiogram as inputs and generates one or more outputs from multiple inputs. Alternatively, the artificial neural network model of the present disclosure may have a 'many-to-many' structure that simultaneously receives multiple types of bio information from among human bio information and simultaneously outputs the expected time of occurrence of different bio events.

The artificial neural network model of the present disclosure may be trained by a multi-task learning method based on a first task of predicting the expected time of occurrence of the bio event and a second task of predicting the probability of occurrence of the bio event. Multi-task learning is a training method for artificial neural networks that allows one model to perform multiple types of tasks that are similar or different from each other. For example, a neural network model may be trained to classify images of dogs and cats from given images as a first task, while at the same time predicting the age of the animal appearing in the image from a given image as a second task.

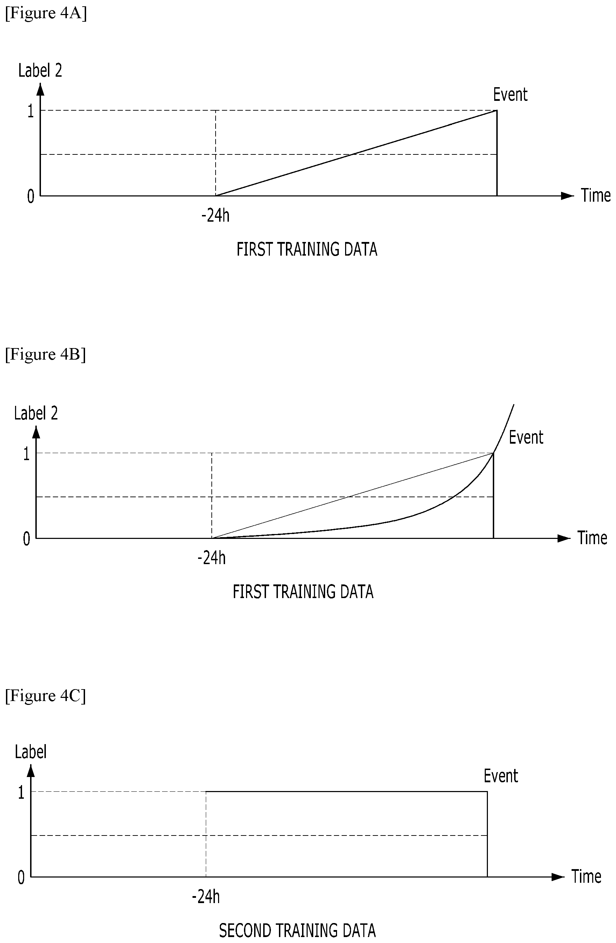

To perform the multi-task learning, the artificial neural network model of the present disclosure may be trained based on two or more different training data sets. At this time, the two or more different training data sets may include second training data, which is bio information with binary information set as a label on whether a bio event occurs within a predetermined time period, and first training data, which is bio information with information on the time of occurrence of a patient's bio event set as a label. A specific description of each training data is provided below with reference to FIGS. 4A to 4C.

By training two different types of training data through the multi-task learning, the artificial neural network model of the present disclosure may simultaneously generate one output predicting the expected time of occurrence of the bio event and another output predicting the probability of occurrence of the bio event. Accordingly, in the present disclosure, the comprehensive prediction information related to the bio event may be provided to the user based on the expected time of occurrence of the bio event and the probability of occurrence of the bio event, and information on the priority or degree of action between patients may be provided to the human physician.

For example, it may be assumed that the artificial neural network model is configured to generate outputs related to cardiac arrest, and that the first output of the artificial neural network model of the present disclosure is 0.8 and the second output is 0.6 for patient A, and that the first output of the artificial neural network is 0.2 and the second output is 0.8 for patient B. At this time, patient B has a higher probability of experiencing cardiac arrest within the predetermined time, but the first output for patient A has a greater value than the first output for patient B. Therefore, it is predicted that the predicted time of cardiac arrest is closer for patient A than for patient B. In this case, in the related art, only the possibility of cardiac arrest is considered, and patient B is given priority over patient A, but it can be seen that according to the artificial neural network model of the present disclosure, even though the possibility of cardiac arrest is low for patient A, the time of occurrence of the bio event is predicted to be much earlier (i.e., sooner) than that of patient B. Therefore, based on the output of the artificial neural network of the present disclosure, a determination may be made to prioritize patient A over patient B.

As another embodiment, the second output may be filtered by comparing the second output with a predetermined threshold, and the priority of the patient may be determined based on the size of the first output for second outputs having a value greater than the threshold. For example, if the threshold is set to 0.8, the second output of patient A in the example above is filtered out because the second output is below the threshold, and the human physician may take priority over patient B in taking action for patient A without having to judge the level of urgency of patient A and patient B.

As another embodiment, the priority may be determined primarily based on the second output, and a final priority may be determined based on the size of the first output. For example, after first classifying patients with the highest priority whose second output has a value from 0.9 to 1, the final priority may be determined based on the value of the first output of those patients. At this time, it will be obvious to those skilled in the art that the range of the second output that determines the priority may be appropriately adjusted according to the various environments in which the present disclosure is implemented.

As a result, the present disclosure has the effect of enabling more appropriate measures to be taken for patients compared to conventional technologies that only provide the possibility of occurrence of the bio event.

FIGS. 4A and 4B are conceptual diagrams illustrating a label of first training data according to an embodiment of the present disclosure.

An artificial neural network model according to an embodiment of the present disclosure may be trained with first training data including bio information with information about a time of occurrence of a bio event set as a label.

The first training data may be bio information data that is assigned continuous or discontinuous label values from 0 to 1 by normalizing the time at which the bio event occurs based on a predetermined time standard. For example, the first training data may be bio information data labeled to have values that linearly increase as the time when the bio event occurs approaches, as illustrated in FIG. 4A. Specifically, if the predetermined time period is 24 hours and a bio event, for example, cardiac arrest, occurs 18 hours later for some training data, the label value of the corresponding training data may be set to 0.25. Further, if cardiac arrest occurs 12 hours later, the label value of the corresponding training data may be set to 0.5. Further, if cardiac arrest occurs 6 hours later for some training data, the label value of the corresponding training data may be set to 0.75.

As another example, the first training data may be bio information data labeled to have values that exponentially grows as the time when the bio event occurs approaches, as illustrated in FIG. 4B.

However, the labeling method in the present disclosure is not limited to the examples illustrated in FIGS. 4A and 4B, and various labeling methods that determine weights in various schemes depending on the time at which the bio event occurs may be used. It will also be apparent to those skilled in the art that the design of the artificial neural network model may optionally be modified to suit the labeling method used.

When trained with such data, the first output of the artificial neural network model that predicts the occurrence time of the bio event may have a value from 0 to 1. Users may interpret that the larger the first output value of the artificial neural network model, the closer the predicted time is for the patient's bio event to occur. In this way, the occurrence time of the bio event may be predicted through model training based on the first training data.

FIG. 4C is a conceptual diagram illustrating a label of second training data according to an embodiment of the present disclosure.

An artificial neural network model according to an embodiment of the present disclosure may be trained with second training data including bio information with "binary information on whether a bio event occurs within a predetermined time" set as a label.

The second training data may include bio data having binary labels, which are labeled as 1 if the bio event occurs within a predetermined time, for example, 24 hours, and 0 otherwise.

The second output of the artificial neural network model that predicts the probability of occurrence of the bio event trained with data having such binary labels may have a real value from 0 to 1. Users may interpret that the larger the second output value of the artificial neural network model, the higher the possibility that the bio event of the patient will occur with a predetermined time period. Therefore, the possibility of the bio event occurring within the predetermined time may be predicted through model training based on the second training data.

In the meantime, according to an embodiment of the present disclosure, a computer readable medium storing a data structure is disclosed.

The data structure may refer to organization, management, and storage of data that enable efficient access and modification of data. The data structure may refer to organization of data for solving a specific problem (for example, data search, data storage, and data modification in the shortest time). The data structure may also be defined with a physical or logical relationship between the data elements designed to support a specific data processing function. A logical relationship between data elements may include a connection relationship between user defined data elements. A physical relationship between data elements may include an actual relationship between the data elements physically stored in a computer readable storage medium (for example, a permanent storage device). In particular, the data structure may include a set of data, a relationship between data, and a function or a command applicable to data. Through the effectively designed data structure, the computing device may perform a calculation while minimally using resources of the computing device. In particular, the computing device may improve efficiency of calculation, reading, insertion, deletion, comparison, exchange, and search through the effectively designed data structure.

The data structure may be divided into a linear data structure and a non-linear data structure according to the form of the data structure. The linear data structure may be the structure in which only one data is connected after one data. The linear data structure may include a list, a stack, a queue, and a deque. The list may mean a series of dataset in which order exists internally. The list may include a linked list. The linked list may have a data structure in which data is connected in a method in which each data has a pointer and is linked in a single line. In the linked list, the pointer may include information about the connection with the next or previous data. The linked list may be expressed as a single linked list, a double linked list, and a circular linked list according to the form. The stack may have a data listing structure with limited access to data. The stack may have a linear data structure that may process (for example, insert or delete) data only at one end of the data structure. The data stored in the stack may have a data structure (Last In First Out, LIFO) in which the later the data enters, the sooner the data comes out. The queue is a data listing structure with limited access to data, and may have a data structure (First In First Out, FIFO) in which the later the data is stored, the later the data comes out, unlike the stack. The deque may have a data structure that may process data at both ends of the data structure.

The non-linear data structure may be the structure in which the plurality of data is connected after one data. The non-linear data structure may include a graph data structure. The graph data structure may be defined with a vertex and an edge, and the edge may include a line connecting two different vertexes. The graph data structure may include a tree data structure. The tree data structure may be the data structure in which a path connecting two different vertexes among the plurality of vertexes included in the tree is one. That is, the tree data structure may be the data structure in which a loop is not formed in the graph data structure.

Throughout the present specification, a calculation model, a nerve network, the network function, and the neural network may be used with the same meaning. Hereinafter, the terms of the calculation model, the nerve network, the network function, and the neural network are unified and described with a neural network. The data structure may include a neural network. Further, the data structure including the neural network may be stored in a computer readable medium. The data structure including the neural network may also include preprocessed data for processing by the neural network, data input to the neural network, a weight of the neural network, a hyper-parameter of the neural network, data obtained from the neural network, an active function associated with each node or layer of the neural network, and a loss function for training of the neural network. The data structure including the neural network may include predetermined configuration elements among the disclosed configurations. That is, the data structure including the neural network may include the entirety or a predetermined combination of pre-processed data for processing by neural network, data input to the neural network, a weight of the neural network, a hyper parameter of the neural network, data obtained from the neural network, an active function associated with each node or layer of the neural network, and a loss function for training the neural network. In addition to the foregoing configurations, the data structure including the neural network may include predetermined other information determining a characteristic of the neural network. Further, the data structure may include all type of data used or generated in a computation process of the neural network, and is not limited to the foregoing matter. The computer readable medium may include a computer readable recording medium and/or a computer readable transmission medium. The neural network may be formed of a set of interconnected calculation units which are generally referred to as "nodes". The "nodes" may also be called "neurons." The neural network consists of one or more nodes.

The data structure may include data input to the neural network. The data structure including the data input to the neural network may be stored in the computer readable medium. The data input to the neural network may include training data input in the training process of the neural network and/or input data input to the training completed neural network. The data input to the neural network may include data that has undergone pre-processing and/or data to be pre-processed. The pre-processing may include a data processing process for inputting data to the neural network. Accordingly, the data structure may include data to be pre-processed and data generated by the pre-processing. The foregoing data structure is merely an example, and the present disclosure is not limited thereto.

The data structure may include a weight of the neural network (in the present specification, weights and parameters may be used with the same meaning), Further, the data structure including the weight of the neural network may be stored in the computer readable medium. The neural network may include a plurality of weights. The weight is variable, and in order for the neural network to perform a desired function, the weight may be varied by a user or an algorithm. For example, when one or more input nodes are connected to one output node by links, respectively, the output node may determine a data value output from the output node based on values input to the input nodes connected to the output node and the weight set in the link corresponding to each of the input nodes. The foregoing data structure is merely an example, and the present disclosure is not limited thereto.

For a non-limited example, the weight may include a weight varied in the neural network training process and/or the weight when the training of the neural network is completed. The weight varied in the neural network training process may include a weight at a time at which a training cycle starts and/or a weight varied during a training cycle. The weight when the training of the neural network is completed may include a weight of the neural network completing the training cycle. Accordingly, the data structure including the weight of the neural network may include the data structure including the weight varied in the neural network training process and/or the weight when the training of the neural network is completed. Accordingly, it is assumed that the weight and/or a combination of the respective weights are included in the data structure including the weight of the neural network. The foregoing data structure is merely an example, and the present disclosure is not limited thereto.

The data structure including the weight of the neural network may be stored in the computer readable storage medium (for example, a memory and a hard disk) after undergoing a serialization process. The serialization may be the process of storing the data structure in the same or different computing devices and converting the data structure into a form that may be reconstructed and used later. The computing device may serialize the data structure and transceive the data through a network. The serialized data structure including the weight of the neural network may be reconstructed in the same or different computing devices through deserialization. The data structure including the weight of the neural network is not limited to the serialization. Further, the data structure including the weight of the neural network may include a data structure (for example, in the non-linear data structure, B-Tree, Trie, m-way search tree, AVL tree, and Red-Black Tree) for improving efficiency of the calculation while minimally using the resources of the computing device. The foregoing matter is merely an example, and the present disclosure is not limited thereto.

The data structure may include a hyper-parameter of the neural network. The data structure including the hyper-parameter of the neural network may be stored in the computer readable medium. The hyper-parameter may be a variable varied by a user. The hyper-parameter may include, for example, a training rate, a cost function, the number of times of repetition of the training cycle, weight initialization (for example, setting of a range of a weight value to be weight-initialized), and the number of hidden units (for example, the number of hidden layers and the number of nodes of the hidden layer). The foregoing data structure is merely an example, and the present disclosure is not limited thereto.

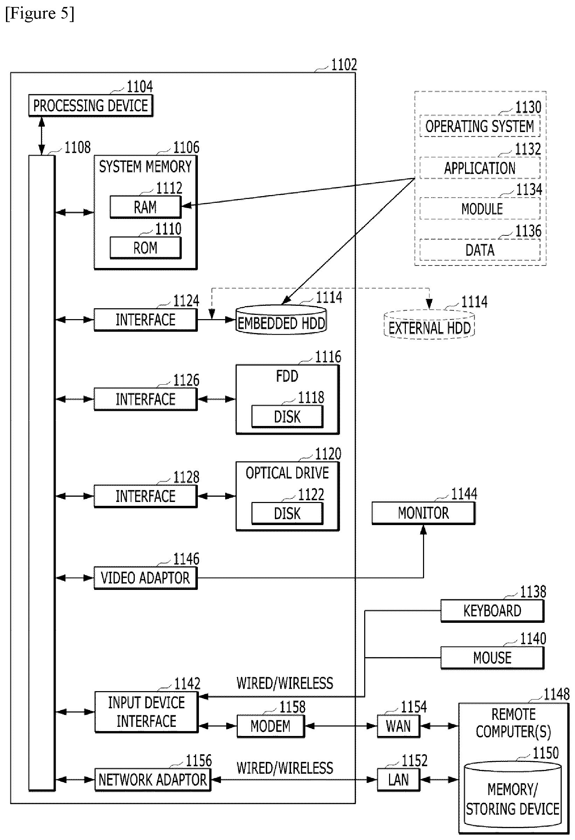

FIG. 5 is a simple and general schematic diagram illustrating an example of a computing environment in which the embodiments of the present disclosure are implementable.

The present disclosure has been described as being generally implementable by the computing device, but those skilled in the art will appreciate well that the present disclosure is combined with computer executable commands and/or other program modules executable in one or more computers and/or be implemented by a combination of hardware and software.

In general, a program module includes a routine, a program, a component, a data structure, and the like performing a specific task or implementing a specific abstract data form. Further, those skilled in the art will well appreciate that the method of the present disclosure may be carried out by a personal computer, a hand-held computing device, a microprocessor-based or programmable home appliance (each of which may be connected with one or more relevant devices and be operated), and other computer system configurations, as well as a single-processor or multiprocessor computer system, a mini computer, and a main frame computer.

The embodiments of the present disclosure may be carried out in a distribution computing environment, in which certain tasks are performed by remote processing devices connected through a communication network. In the distribution computing environment, a program module may be located in both a local memory storage device and a remote memory storage device.

The computer generally includes various computer readable media. The computer accessible medium may be any type of computer readable medium, and the computer readable medium includes volatile and non-volatile media, transitory and non-transitory media, and portable and non-portable media. As a non-limited example, the computer readable medium may include a computer readable storage medium and a computer readable transport medium. The computer readable storage medium includes volatile and non-volatile media, transitory and non-transitory media, and portable and non-portable media constructed by a predetermined method or technology, which stores information, such as a computer readable command, a data structure, a program module, or other data. The computer readable storage medium includes a RAM, a Read Only Memory (ROM), an Electrically Erasable and Programmable ROM (EEPROM), a flash memory, or other memory technologies, a Compact Disc (CD)-ROM, a Digital Video Disk (DVD), or other optical disk storage devices, a magnetic cassette, a magnetic tape, a magnetic disk storage device, or other magnetic storage device, or other predetermined media, which are accessible by a computer and are used for storing desired information, but is not limited thereto.

The computer readable transport medium generally implements a computer readable command, a data structure, a program module, or other data in a modulated data signal, such as a carrier wave or other transport mechanisms, and includes all of the information transport media. The modulated data signal means a signal, of which one or more of the characteristics are set or changed so as to encode information within the signal. As a non-limited example, the computer readable transport medium includes a wired medium, such as a wired network or a direct-wired connection, and a wireless medium, such as sound, Radio Frequency (RF), infrared rays, and other wireless media. A combination of the predetermined media among the foregoing media is also included in a range of the computer readable transport medium.

An illustrative environment 1100 including a computer 1102 and implementing several aspects of the present disclosure is illustrated, and the computer 1102 includes a processing device 1104, a system memory 1106, and a system bus 1108. The system bus 1108 connects system components including the system memory 1106 (not limited) to the processing device 1104. The processing device 1104 may be a predetermined processor among various commonly used processors. A dual processor and other multiprocessor architectures may also be used as the processing device 1104.

The system bus 1108 may be a predetermined one among several types of bus structure, which may be additionally connectable to a local bus using a predetermined one among a memory bus, a peripheral device bus, and various common bus architectures. The system memory 1106 includes a ROM 1110, and a RAM 1112. A basic input/output system (BIOS) is stored in a non-volatile memory 1110, such as a ROM, an EPROM, and an EEPROM, and the BIOS includes a basic routing helping a transport of information among the constituent elements within the computer 1102 at a time, such as starting. The RAM 1112 may also include a high-rate RAM, such as a static RAM, for caching data.

The computer 1102 also includes an embedded hard disk drive (HDD) 1114 (for example, enhanced integrated drive electronics (EIDE) and serial advanced technology attachment (SATA)) - the embedded HDD 1114 being configured for exterior mounted usage within a proper chassis (not illustrated) - a magnetic floppy disk drive (FDD) 1116 (for example, which is for reading data from a portable diskette 1118 or recording data in the portable diskette 1118), and an optical disk drive 1120 (for example, which is for reading a CD-ROM disk 1122, or reading data from other high-capacity optical media, such as a DVD, or recording data in the high-capacity optical media). A hard disk drive 1114, a magnetic disk drive 1116, and an optical disk drive 1120 may be connected to a system bus 1108 by a hard disk drive interface 1124, a magnetic disk drive interface 1126, and an optical drive interface 1128, respectively. An interface 1124 for implementing an outer mounted drive includes, for example, at least one of or both a universal serial bus (USB) and the Institute of Electrical and Electronics Engineers (IEEE) 1394 interface technology.

The drives and the computer readable media associated with the drives provide non-volatile storage of data, data structures, computer executable commands, and the like. In the case of the computer 1102, the drive and the medium correspond to the storage of random data in an appropriate digital form. In the description of the computer readable media, the HDD, the portable magnetic disk, and the portable optical media, such as a CD, or a DVD, are mentioned, but those skilled in the art will well appreciate that other types of computer readable media, such as a zip drive, a magnetic cassette, a flash memory card, and a cartridge, may also be used in the illustrative operation environment, and the predetermined medium may include computer executable commands for performing the methods of the present disclosure.

A plurality of program modules including an operation system 1130, one or more application programs 1132, other program modules 1134, and program data 1136 may be stored in the drive and the RAM 1112. An entirety or a part of the operation system, the application, the module, and/or data may also be cached in the RAM 1112. It will be well appreciated that the present disclosure may be implemented by several commercially usable operation systems or a combination of operation systems.

A user may input a command and information to the computer 1102 through one or more wired/wireless input devices, for example, a keyboard 1138 and a pointing device, such as a mouse 1140. Other input devices (not illustrated) may be a microphone, an IR remote controller, a joystick, a game pad, a stylus pen, a touch screen, and the like. The foregoing and other input devices are frequently connected to the processing device 1104 through an input device interface 1142 connected to the system bus 1108, but may be connected by other interfaces, such as a parallel port, an IEEE 1394 serial port, a game port, a USB port, an IR interface, and other interfaces.

A monitor 1144 or other types of display devices are also connected to the system bus 1108 through an interface, such as a video adaptor 1146. In addition to the monitor 1144, the computer generally includes other peripheral output devices (not illustrated), such as a speaker and a printer.

The computer 1102 may be operated in a networked environment by using a logical connection to one or more remote computers, such as remote computer(s) 1148, through wired and/or wireless communication. The remote computer(s) 1148 may be a work station, a computing device computer, a router, a personal computer, a portable computer, a microprocessor-based entertainment device, a peer device, and other general network nodes, and generally includes some or an entirety of the constituent elements described for the computer 1102, but only a memory storage device 1150 is illustrated for simplicity. The illustrated logical connection includes a wired/wireless connection to a local area network (LAN) 1152 and/or a larger network, for example, a wide area network (WAN) 1154. The LAN and WAN networking environments are general in an office and a company, and make an enterprise-wide computer network, such as an Intranet, easy, and all of the LAN and WAN networking environments may be connected to a worldwide computer network, for example, the Internet.

When the computer 1102 is used in the LAN networking environment, the computer 1102 is connected to the local network 1152 through a wired and/or wireless communication network interface or an adaptor 1156. The adaptor 1156 may make wired or wireless communication to the LAN 1152 easy, and the LAN 1152 also includes a wireless access point installed therein for the communication with the wireless adaptor 1156. When the computer 1102 is used in the WAN networking environment, the computer 1102 may include a modem 1158, is connected to a communication computing device on a WAN 1154, or includes other means setting communication through the WAN 1154 via the Internet. The modem 1158, which may be an embedded or outer-mounted and wired or wireless device, is connected to the system bus 1108 through a serial port interface 1142. In the networked environment, the program modules described for the computer 1102 or some of the program modules may be stored in a remote memory/storage device 1150. The illustrated network connection is illustrative, and those skilled in the art will appreciate well that other means setting a communication link between the computers may be used.

The computer 1102 performs an operation of communicating with a predetermined wireless device or entity, for example, a printer, a scanner, a desktop and/or portable computer, a portable data assistant (PDA), a communication satellite, predetermined equipment or place related to a wirelessly detectable tag, and a telephone, which is disposed by wireless communication and is operated. The operation includes a wireless fidelity (Wi-Fi) and Bluetooth wireless technology at least. Accordingly, the communication may have a pre-defined structure, such as a network in the related art, or may be simply ad hoc communication between at least two devices.

The Wi-Fi enables a connection to the Internet and the like even without a wire. The Wi-Fi is a wireless technology, such as a cellular phone, which enables the device, for example, the computer, to transmit and receive data indoors and outdoors, that is, in any place within a communication range of a base station. A Wi-Fi network uses a wireless technology, which is called IEEE 802.11 (a, b, g, etc.) for providing a safe, reliable, and high-rate wireless connection. The Wi-Fi may be used for connecting the computer to the computer, the Internet, and the wired network (IEEE 802.3 or Ethernet is used). The Wi-Fi network may be operated at, for example, a data rate of 11 Mbps (802.11a) or 54 Mbps (802.11b) in an unauthorized 2.4 and 5 GHz wireless band, or may be operated in a product including both bands (dual bands).

Those skilled in the art may appreciate that information and signals may be expressed by using predetermined various different technologies and techniques. For example, data, indications, commands, information, signals, bits, symbols, and chips referable in the foregoing description may be expressed with voltages, currents, electromagnetic waves, magnetic fields or particles, optical fields or particles, or a predetermined combination thereof.

Those skilled in the art will appreciate that the various illustrative logical blocks, modules, processors, means, circuits, and algorithm operations described in relationship to the embodiments disclosed herein may be implemented by electronic hardware (for convenience, called "software" herein), various forms of program or design code, or a combination thereof. In order to clearly describe compatibility of the hardware and the software, various illustrative components, blocks, modules, circuits, and operations are generally illustrated above in relation to the functions of the hardware and the software. Whether the function is implemented as hardware or software depends on design limits given to a specific application or an entire system. Those skilled in the art may perform the function described by various schemes for each specific application, but it shall not be construed that the determinations of the performance depart from the scope of the present disclosure.

Various embodiments presented herein may be implemented by a method, a device, or a manufactured article using a standard programming and/or engineering technology. A term "manufactured article" includes a computer program, a carrier, or a medium accessible from a predetermined computer-readable storage device. For example, the computer-readable storage medium includes a magnetic storage device (for example, a hard disk, a floppy disk, and a magnetic strip), an optical disk (for example, a CD and a DVD), a smart card, and a flash memory device (for example, an EEPROM, a card, a stick, and a key drive), but is not limited thereto. Further, various storage media presented herein include one or more devices and/or other machine-readable media for storing information.

It shall be understood that a specific order or a hierarchical structure of the operations included in the presented processes is an example of illustrative accesses. It shall be understood that a specific order or a hierarchical structure of the operations included in the processes may be rearranged within the scope of the present disclosure based on design priorities. The accompanying method claims provide various operations of elements in a sample order, but it does not mean that the claims are limited to the presented specific order or hierarchical structure.

The description of the presented embodiments is provided so as for those skilled in the art to use or carry out the present disclosure. Various modifications of the embodiments may be apparent to those skilled in the art, and general principles defined herein may be applied to other embodiments without departing from the scope of the present disclosure. Accordingly, the present disclosure is not limited to the embodiments suggested herein, and shall be interpreted within the broadest meaning range consistent to the principles and new characteristics presented herein.

### [Mode for Invention]

Related contents in the best mode for carrying out the present disclosure are described as above.

## Claims

1. A method performed by a computing device to predict a bio event, the method comprising:
obtaining bio information of a patient; and
generating a first output indicating an expected time of occurrence of the bio event based on the bio information by using an artificial neural network model.

2. The method of claim 1, wherein the bio event includes an event related to a disease determined to affect a health status of the patient.

3. The method of claim 1, further comprising:
generating a second output indicating a possibility of occurrence of the bio event within a predetermined time period by using the artificial neural network model.

4. The method of claim 1, wherein the first output includes a value corresponding to a length of a remaining time from a time when the bio information is obtained up to a time when the bio event is predicted to occur.

5. The method of claim 3, further comprising:
providing comprehensive prediction information related to the bio event based on the first output and the second output to a user,
wherein the comprehensive prediction information includes information capable of determining a priority for each patient among all patients.

6. The method of claim 5, wherein the comprehensive prediction information includes information filtered through a comparison between the second output and a predetermined threshold, and priority information of the patient determined based on the filtered result and a size of the first output.

7. A method performed by a computing device to predict a bio event, the method comprising:
obtaining bio information of a patient; and
generating prediction information related to a bio event based on the bio information by using an artificial neural network model,
wherein the artificial neural network model is a model trained by multi-task learning based on a first task of predicting an expected time of occurrence of the bio event and a second task of predicting a probability of occurrence of the bio event.

8. The method of claim 7, wherein the artificial neural network model is trained based on two or more different training data sets.

9. The method of claim 8, wherein the two or more different training data sets include first training data which is bio information with information on an occurrence time of the bio event of the patient set as a label.

10. The method of claim 8, wherein the two or more different training data sets include second training data which is bio information with binary information indicating whether the bio event occurs within a predetermined time period set as a label.

11. A computer program stored in a non-transitory computer readable storage medium, wherein the computer program includes instructions which allow operations of predicting a bio event to be performed, the operations comprise:
an operation of obtaining bio information of a patient; and
an operation of generating a first output indicating an expected time of occurrence of the bio event based on the bio information by using an artificial neural network model.

12. A computing device for predicting a bio event, comprising:
at least one processor; and
a memory,
wherein the at least one processor is configured to:
obtain bio information of a patient, and
generate a first output indicating an expected time of occurrence of the bio event based on the bio information by using an artificial neural network model.
